(19) **Europäisches Patentamt / European Patent Office / Office européen des brevets**

(11) **EP 2 607 432 A1**

(12) **EUROPÄISCHE PATENTANMELDUNG**

(43) Veröffentlichungstag:
26.06.2013  Patentblatt 2013/26

(51) Int Cl.:
*C09C 1/00* *(2006.01)*

(21) Anmeldenummer: 12007941.3

(22) Anmeldetag: 26.11.2012

(84) Benannte Vertragsstaaten:
AL AT BE BG CH CY CZ DE DK EE ES FI FR GB
GR HR HU IE IS IT LI LT LU LV MC MK MT NL NO
PL PT RO RS SE SI SK SM TR
Benannte Erstreckungsstaaten:
BA ME

(30) Priorität: 21.12.2011  DE 102011121804
19.01.2012  DE 102012000887

(71) Anmelder: Merck Patent GmbH
64293 Darmstadt (DE)

(72) Erfinder:
• Pfaff, Gerhard
  64839 Muenster (DE)
• Andes, Stephanie
  63452 Hanau (DE)
• Ambrosius, Klaus
  64807 Dieburg (DE)
• Petry, Ralf
  64347 Griesheim (DE)
• Roesler, Michael
  64354 Reinheim (DE)
• Schoen, Sabine
  45701 Herten (DE)

(54) **Effektpigmente basierend auf Substraten die einen Kreisformfaktor von 1,2-2 aufweisen**

(57)    Die vorliegende Erfindung betrifft Effektpigmente, die auf plättchenförmigen Substraten mit einem Kreisformfaktor von 1,2 - 2 basieren und mit mindestens einer hochbrechenden Schicht beschichtet sind, sowie deren Verwendung, u.a. in Farben, Lacken, Druckfarben, Kunststoffen und in kosmetischen Formulierungen.

EP 2 607 432 A1

**Beschreibung**

Die vorliegende Erfindung betrifft Effektpigmente, die auf plättchenförmigen Substraten mit einem Kreisformfaktor von 1,2 - 2 basieren und mit mindestens einer hochbrechenden Schicht beschichtet sind, sowie deren Verwendung, u.a. in Farben, Lacken, Druckfarben, Kunststoffen, und in kosmetischen Formulierungen.

Effektpigmente, wie z.B. Perlglanzpigmente oder Metalleffektpigmente, werden in vielen Bereichen der Technik eingesetzt, insbesondere im Bereich der Autolacke, Industrielacke, der dekorativen Beschichtung, im Kunststoff, in Farben, Druckfarben sowie in kosmetischen Formulierungen. Derartige Pigmente basieren auf plättchenförmigen länglichen Substraten, die ein- oder mehrfach beschichtet sind.

Die Größe der Basissubstrate ist bei den Effektpigmenten in der Regel an sich nicht kritisch und kann auf den jeweiligen Anwendungszweck abgestimmt werden. In der Regel haben die plättchenförmigen und länglichen Substrate eine Dicke zwischen 0,1 und 5 $\mu$m, insbesondere zwischen 0,2 und 4,5 $\mu$m. Die Ausdehnung in den beiden anderen Bereichen beträgt üblicherweise zwischen 1 und 250 $\mu$m, vorzugsweise zwischen 2 und 200 $\mu$m und insbesondere zwischen 5 und 60 $\mu$m. Die im Markt angebotenen Effektpigmente zeichnen sich in der Regel durch eine breite Teilchengrößen- und Dickenverteilung aus.

Die optischen Eigenschaften der Effektpigmente, wie z.B. Farbe und Farbflop (also Farbtonwinkel, Sättigung und Helligkeit auch ihrer winkelabhängigen Ausprägung) werden in entscheidendem Maße durch die Brechungsindices der Interferenzschichten auf den Substratplättchen sowie deren geometrische Dicke bestimmt. Effektpigmente haben aber in der Regel den Nachteil, dass sie ein zu geringes Deckvermögen und eine zu geringe Farbsättigung aufweisen.

Aufgabe der vorliegenden Erfindung ist es, Effektpigmente zu finden, die ohne Verlust ihrer optischen Eigenschaften, insbesondere des Glanzes und der Farbreinheit, eine hohe Farbsättigung aufweisen und gleichzeitig ein hohes Deckvermögen zeigen und sich durch vorteilhafte Anwendungseigenschaften auszeichnen.

Überraschenderweise wurde nun gefunden, dass Effektpigmente basierend auf plättchenförmigen Substraten, die eine rundliche Form aufweisen gegenüber den Effektpigmenten aus dem Stand der Technik basierend auf Substraten mit einer länglichen, einer höheren Kantenrauhigkeit besitzende, Form, eine erhöhte Farbsättigung und ein erhöhtes Deckvermögen aufzeigen.

Gegenstand der vorliegenden Erfindung sind daher Effektpigmente, die auf plättchenförmigen Substraten basieren, wobei die Substrate einen Kreisformfaktor (Umfang$^2$ / Fläche normiert auf einen Kreis) von 1,2 - 2 aufweisen, und mit mindestens einer hochbrechenden Schicht mit einem Brechungsindex von n $\geq$ 1,8 beschichtet sind.

Unter Kreisformfaktor wird in dieser Anmeldung das Verhältnis des Umfangquadrates zur Fläche des im lichtmikroskopischen Durchlicht mit 30facher Vergrößerung abgebildeten Einzelpartikels festgelegt. Zur Vereinfachung wird das Resultat durch $4\pi$ dividiert, woraus sich dann für den Kreisformfaktor des idealen Kreises 1 ergibt. Die ausgewerteten Partikel liegen weitgehend eben in der Abbildungsebene und die Anzahl der ausgewerteten Partikel ist hinreichend statistisch aussagekräftig (N = 2000) für den Kreisformfaktor-Mittelwert.

Gegenstand der Erfindung ist weiterhin die Verwendung der erfindungsgemäßen Pigmente in Farben, Autolacken, Industrielacken, Lacken, Druckfarben, Kunststoffen, Knopfpasten, keramischen Materialien, Gläsern, zur Saatguteinfärbung, als Absorber bei der Lasermarkierung von Kunststoffen, Gläsern, Pappen und Papieren, als Absorber beim Laserschweißen von Kunststoffen, als Additiv zur Farbgebung von Lebensmittel- und Pharmaprodukten, als Additiv zur Farbgebung von Überzügen von Lebensmittel- und Pharmaprodukten, in kosmetischen Formulierungen, zur Herstellung von Pigmentpräparationen und Trockenpräparaten und bei fälschungssicheren Wertpapieren. Geeignete Basissubstrate für die erfindungsgemäßen Effektpigmente sind transparente plättchenförmige Substrate. Bevorzugte Substrate sind Schichtsilikate, wie z. B. natürlicher oder synthetische Glimmer, Talkum, Kaolin, Graphit, plättchenförmige Eisenoxide, Glas-, SiO$_2$-, Al$_2$O$_3$-, TiO$_2$- oder synthetische Keramikplättchen, synthetische trägerfreie Plättchen, LCPs (Liquid Crystal Polymers) oder andere vergleichbare Materialien. Ganz besonders bevorzugte Substratplättchen sind natürliche oder synthetische Glimmerplättchen, Glasplättchen, Al$_2$O$_3$-Plättchen und SiO$_2$-Plättchen.

Für die erfindungsgemäßen Effektpigmente werden Substratplättchen mit einem Kreisformfaktor von 1,2 - 2, vorzugsweise 1,2 - 1,8, und ganz besonders bevorzugt 1,2 - 1,7, eingesetzt.

Bevorzugte Substratplättchen besitzen eine Partikelgröße von 5-60 $\mu$m, insbesondere von 5-40 $\mu$m. Die Dicke der bevorzugten Substratplättchen beträgt vorzugsweise 0,2 - 0,6 $\mu$m.

In der vorliegenden Patentanmeldung werden die Partikelgrößen mit Hilfe des Mastersizer 2000 der Fa. Malvern UK bestimmt.

Die Substratplättchen können beispielsweise wie folgt hergestellt werden:

Unter Verwendung von bekannten mechanischen Zerkleinerungsverfahren, werden Substratbrocken, z.B. durch Mahlen, zerkleinert und delaminiert und entsprechend den Anforderungen in Bezug auf Äquivalenzdurchmesser und Dicken der Plättchen mittels Sedimentation, Dekantierung, Windsichtung und/oder Siebung klassiert.

Für das Mahlverfahren kommen alle dem Fachmann bekannten Mühlen und Rührer, zum Einsatz, insbesondere alle schnell laufenden Rührer, Dispergatoren oder Rotor-Stator-Mühlen.

Die Substratplättchen, beispielsweise Glimmer- oder Glasplättchen, werden hergestellt, indem größere Brocken oder grobe Flocken gemahlen werden. Anschließend werden die entstehenden Substratplättchen in der Regel mit einem Durchmesser von 50-200 µm in ein Zerkleinerungsaggregat, z. B. eine Rotor-Stator-Mühle, gegeben und mit Wasser und/oder einem organischen Lösemittel, vorzugsweise Wasser, versetzt. Die so entstehende Suspension wird über mehrere Stunden in dem Zerkleinerungsaggregat mechanisch behandelt; wobei gleichzeitig die Oberfläche der Plättchen glatt poliert wird. Die mechanische Belastung der Teilchen während dieses Schrittes wird dabei so gewählt, dass eine permanente Scherung zur weiteren schonenden Delaminierung von Teilchen und zur Glättung der Kanten und Oberflächen führt. Eine enge Teilchengrößenverteilung wird durch einen anschließenden Klassierschritt in Form von mehreren, mindestens ≥ 2, vorzugsweise ≥ 3, Sedimentationsstufen erreicht. Die so erzeugten dünnen und rundlichen Plättchen weisen eine Teilchengrößenverteilung von 5-60 µm, eine Dickenverteilung von 0,2 - 0,6 µm sowie glatt polierte Oberflächen mit nur wenigen scharfen Kanten auf. Der Kreisformfaktor beträgt 1,2-2.

Während der Mahlung und Klassierung werden kleinere, dickere Partikel und größere, dünnere Partikel entfernt, d.h. große Partikel werden eher nur zerbrochen, während kleinere Partikel delaminiert und/oder herausklassiert werden.

Durch die zusätzliche mechanische Behandlung und den damit verbundenen Poliereffekt wird eine Glättung der Oberfläche der Substratpartikel erreicht, wobei außerdem die längliche Plättchenform in eine rundliche Plättchenform überführt wird. Die Abnahme des Kreisformfaktors und der Länglichkeit geht damit einher, dass die Kanten der Substrate glatter werden und die Substrate eine rundlichere Form annehmen.

Künstlich hergestellte Substrate, die als Basissubstrat für Effektpigmente dienen sollen und nicht in Form grober Brocken oder Flocken vorliegen, wie z.B. $Al_2O_3$, $SiO_2$, $Fe_2O_3$, $TiO_2$, Glas, werden direkt dem Zerkleinerungsaggregat, z. B. eine Rotor-Stator-Mühle, zur Zerkleinerung und Polierung der Oberfläche zugeführt. Der Kreisformfaktor beträgt auch hier 1,2-2.

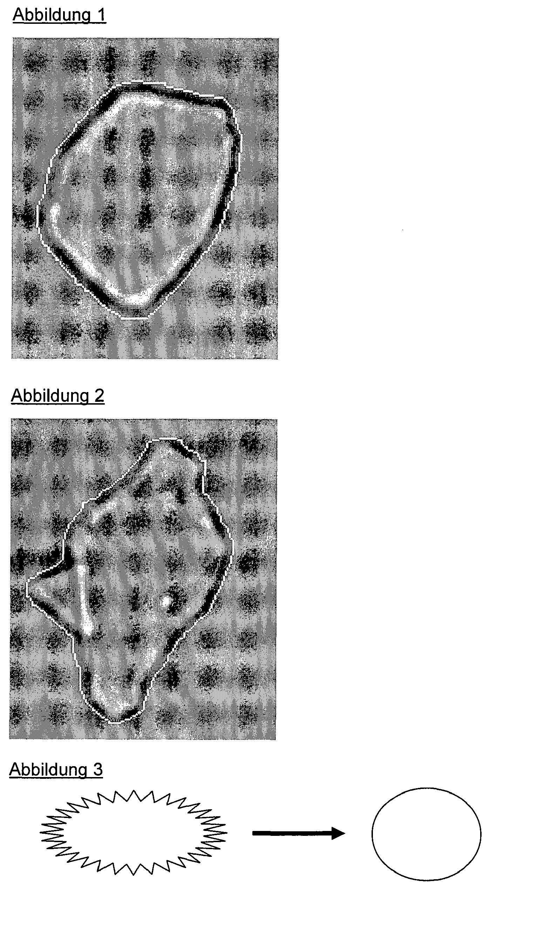

Die mikroskopischen Aufnahmen (Abb. 1 und 2: Bildausschnitt der Größe 43 x 35 µm) zeigen, dass die rundlichen Substratplättchen (Abbildung 1) im Vergleich zu den länglichen Substratplättchen (Abbildung 2) eine deutlich geringere Kantenrauhigkeit aufweisen, wie in Abbildung 3 schematisch dargestellt.

Die geringere Kantenrauhigkeit und die Verminderung der Stufen auf den Plättchenoberflächen generell, führen dazu, dass man bei der Belegung der Substratplättchen sehr gleichmäßige Beschichtungen erhält. Dies betrifft einerseits die lokale Homogenität der Interferenzfarbe als auch die Reduzierung von Streulichteinflüssen, wodurch insgesamt eine höhere Farbsättigung erreicht wird, verglichen mit den Effektpigmenten aus dem Stand der Technik.

Die rundlichen Substratplättchen können als Füllstoff, insbesondere als kosmetischer Füllstoff, oder als Basissubstrat zur Erzeugung von Effektpigmenten dienen. In diesem Fall werden die rundlichen Substratplättchen mit ein oder mehreren Beschichtungen, vorzugsweise Metalloxidschichten, versehen.

Vorzugsweise werden die rundlichen Substratplättchen mit mindestens einer hochbrechenden Schicht (n ≥ 1,8) belegt.

Häufig empfiehlt es sich bei gegenüber Säuren und/oder Basen empfindlichen Substratplättchen, wie z.B. Glasplättchen, diese vor der eigentlichen Belegung zunächst mit einer dünnen Schutzschicht zu überziehen um beispielsweise eine Auslaugung und/oder Quellung der Substrate während der Belegung zu verhindern. Die Schutzschicht kann aber auch dazu dienen eine noch glattere Substratoberfläche zu erzielen. Diese Schutzschicht ist in der Regel sehr dünn, vorzugsweise < 20 nm und hat damit keinen oder nur einen äußerst geringen Einfluss auf die optischen Eigenschaften des finalen Effektpigments. Bei der Schutzschicht handelt es sich vorzugsweise um eine Schicht aus $SiO_2$.

Als hochbrechend wird in dieser Patentanmeldung ein Brechungsindex von n ≥ 1,8, vorzugsweise n ≥ 2,0 verstanden. Als niedrigbrechend wird in dieser Patentanmeldung ein Brechungsindex von n < 1,8 verstanden.

Als Schichtmaterial eignen sich alle dem Fachmann bekannten hochbrechenden Materialien, die filmartig und dauerhaft auf die Substratteilchen aufgebracht werden können, wie z.B. Metalloxide, Metalloxidgemische, Metalloxyhydate, Metallsulfide, Eisentitanate, Eisenoxidhydrate, Titansuboxide, Metalle, sowie Mischungen oder Mischphasen der genannten Verbindungen. Besonders geeignet sind Metalloxide oder Metalloxidgemische, wie z.B. $TiO_2$, $BiOCl$, $Ce_2O_3$, $Cr_2O_3$, $CoO$, $Co_3O_4$, $Fe_2O_3$, $Fe_3O_4$, $FeOOH$, $NiO$, $SnO_2$, $VO_2$, $V_2O_3$, $ZrO_2$, $ZnO$, $CoAl_2O_4$, $BiVO_4$, Eisentitanate, Eisenoxidhydrate, Titansuboxide ($TiO_2$ teilweise reduziert mit Oxidationszahlen von < 4 bis 2 wie $Ti_3O_5$, $Ti_2O_3$ bis zu $TiO$), sowie Metallsulfide, wie z.B. $Ce_2S_3$, $MoS_2$ sowie Mischungen bzw. Mischphasen der genannten Verbindungen untereinander oder mit anderen Metalloxiden sowie Metalle, wie z.B. Aluminium, Chrom, Nickel, Silber, Gold, Titan, Kupfer oder deren Legierungen.

Sofern die Substratplättchen mit einer, zwei oder mehr hochbrechenden Schichten direkt auf der Oberfläche des Substrats belegt sind, handelt es sich vorzugsweise um eine Schicht aus $TiO_2$, $Fe_2O_3$ oder ein Gemisch aus $TiO_2/Fe_2O_3$ oder eine Belegung von $TiO_2$ gefolgt von einer $Fe_2O_3$-Schicht.

Mehrschichtpigmente mit mindestens drei Schichten weisen vorzugsweise eine alternierende Belegung von hoch- und niedrigbrechenden Schichten auf. Besonders bevorzugt ist ein Dreischichtsystem auf dem Substratplättchen mit der Belegung hochbrechend - niedrigbrechend - hochbrechend, wie z.B. eine Belegung mit $TiO_2$ - $SiO_2$ - $TiO_2$.

Die Dicke der hochbrechenden Schicht beträgt in der Regel 20 - 500 nm, vorzugsweise 30 - 400 nm und insbesondere 40 - 350 nm.

Farblose niedrigbrechende für die Beschichtung geeignete Materialien sind vorzugsweise Metalloxide bzw. die entsprechenden Oxidhydrate, wie z.B. $SiO_2$, $Al_2O_3$, $AlO(OH)$, $B_2O_3$, ferner $MgF_2$, $MgSiO_3$ oder ein Gemisch der genannten Verbindungen. Vorzugsweise besteht die niedrigbrechende Schicht aus $SiO_2$, $Al_2O_3$ oder $MgF_2$, insbesondere aus $SiO_2$.

Die Dicke der niedrigbrechenden Schicht beträgt vorzugsweise 10 - 200 nm, insbesondere 10-80 nm und ganz besonders bevorzugt 20-80 nm.

Die Effektpigmente können neben den genannten hoch- und niedrigbrechenden Schichten zusätzlich mit einer absorbierenden Schicht als finale Schicht belegt werden. Vorzugsweise werden die Effektpigmente dann mit Berliner Blau, Karminrot, Thioindigo oder Chromoxid belegt.

Die absorbierende Schicht weist vorzugsweise Schichtdicken von 3-300 nm auf.

Zur Herstellung von silberweißen Effektpigmenten, wie z.B. beschrieben in EP 1865032 A2, empfiehlt es sich die Substratplättchen mit einer hochbrechenden Schicht zu belegen, die neben Titandioxid mindestens eine schwerlösliche Erdalkalimetallverbindung enthält. Der Brechungsindex dieser Schicht ist $\geq 1,9$, vorzugsweise $\geq 2,0$ und insbesondere $\geq 2,1$. Diese hochbrechende Beschichtung kann aus einem Gemisch aus $TiO_2$ und einer schwerlöslichen Erdalkalimetallverbindung und/oder Zinkoxid bestehen oder aus zwei separaten Schichten. In diesem Fall wird auf die $TiO_2$-Schicht eine dünne Schicht aus einer schwerlöslichen Erdalkalimetallverbindung bzw. Zinkoxid aufgebracht.

Das Titandioxid kann in der hochbrechenden Beschichtung in der Rutil- oder in der Anatasmodifikation vorliegen, vorzugsweise liegt es als Rutil vor. Die Verfahren zur Herstellung von Rutil sind im Stand der Technik beispielsweise beschrieben in der U.S. 5,433,779, U.S. 4,038,099, U.S. 6,626,989, DE 25 22 572 C2, EP 0 271 767 B1. Vorzugsweise wird vor der $TiO_2$-Auffällung auf das Substratplättchen, vorzugsweise ein natürliches oder synthetisches Glimmerplättchen, eine dünne Zinndioxidschicht (< 10 nm) aufgebracht, die als Additiv dient um das $TiO_2$ als Rutilphase zu erhalten.

Bevorzugte erfindungsgemäße Effektpigmente besitzen folgende Beschichtung direkt auf der Oberfläche des Substratplättchens:

Substratplättchen + $TiO_2$ (Anatas)
Substratplättchen + $SnO_2$ + $TiO_2$ (Rutil)
Substratplättchen + $TiO_2$ + Erdalkalititanat
Substratplättchen + $SnO_2$ + $TiO_2$ + Erdalkalititanat
Substratplättchen + $TiO_2$ + $Fe_2O_3$
Substratplättchen + $SnO_2$ + $TiO_2$ + $Fe_2O_3$
Substratplättchen + $TiO_2$ + $Fe_3O_4$
Substratplättchen + $SnO_2$ + $TiO_2$ + $Fe_3O_4$
Substratplättchen + $TiO_2$ + $Cr_2O_3$
Substratplättchen + $SnO_2$ + $TiO_2$ + $Cr_2O_3$
Substratplättchen + $TiO_2$ + Karminrot
Substratplättchen + $SnO_2$ + $TiO_2$ + Karminrot
Substratplättchen + $TiO_2$ + Berliner Blau
Substratplättchen + $SnO_2$ + $TiO_2$ + Berliner Blau
Substratplättchen + $TiO_2$ / $Fe_2O_3$
Substratplättchen + $SnO_2$ + $TiO_2$ / $Fe_2O_3$
Substratplättchen + $TiO_2$ + $SiO_2$ + $TiO_2$
Substratplättchen + $SnO_2$ + $TiO_2$ + $SiO_2$ + $SnO_2$ + $TiO_2$
Substratplättchen + $Fe_2O_3$
Substratplättchen + $Fe_3O_4$
Substratplättchen + $TiO_2$ + $SiO_2$ + $TiO_2/Fe_2O_3$
Substratplättchen + $SnO_2$ + $TiO_2$ + $SiO_2$ + $TiO_2/Fe_2O_3$
Substratplättchen + $TiO_2$ + $SiO_2$ + $TiO_2$ + $TiO_2/Fe_2O_3$
Substratplättchen + $SnO_2$ + $TiO_2$ + $SiO_2$ + $TiO_2$ + $TiO_2/Fe_2O_3$
Substratplättchen + $SnO_2$ + $TiO_2$ + $SiO_2$ + $SnO_2$ + $TiO_2$ + $TiO_2/Fe_2O_3$
Substratplättchen + $TiO_2/Fe_2O_3$ + $SiO_2$ + $TiO_2$ + $TiO_2/Fe_2O_3$
Substratplättchen + $TiO_2/Fe_2O_3$ + $SiO_2$ + $SnO_2$ + $TiO_2$ + $TiO_2/Fe_2O_3$
Substratplättchen + $TiO_2/Fe_2O_3$ + $SiO_2$ + $TiO_2$
Substratplättchen + $TiO_2/Fe_2O_3$ + $SiO_2$ + $SnO_2$ + $TiO_2$
Substratplättchen + $TiO_2/Fe_2O_3$ + $SiO_2$ + $TiO_2/Fe_2O_3$
Substratplättchen + $TiO_2$ + $SiO_2$ + $Fe_3O_4$
Substratplättchen + $TiO_2$ + $SiO_2$+ $Cr_2O_3$
Substratplättchen + $Cr_2O_3$
Substratplättchen + Ag
Substratplättchen + Au

Substratplättchen + $Fe_2O_3$ + $SiO_2$ + $TiO_2$/$Fe_2O_3$
Substratplättchen + $SiO_2$ + $TiO_2$ (Anatas)
Substratplättchen + $SiO_2$ + $SnO_2$ + $TiO_2$ (Rutil)
Substratplättchen + $SiO_2$ + $TiO_2$ + Erdalkalititanat
Substratplättchen + $SiO_2$ + $SnO_2$ + $TiO_2$ + Erdalkalititanat
Substratplättchen + $SiO_2$ + $TiO_2$ + $Fe_2O_3$
Substratplättchen + $SiO_2$ + $SnO_2$ + $TiO_2$ + $Fe_2O_3$
Substratplättchen + $SiO_2$ + $TiO_2$ + $Fe_3O_4$
Substratplättchen + $SiO_2$ + $SnO_2$ + $TiO_2$ + $Fe_3O_4$
Substratplättchen + $SiO_2$ + $TiO_2$ + $Cr_2O_3$
Substratplättchen + $SiO_2$ + $SnO_2$ + $TiO_2$ + $Cr_2O_3$
Substratplättchen + $SiO_2$ + $TiO_2$ + Karminrot
Substratplättchen + $SiO_2$ + $SnO_2$ + $TiO_2$ + Karminrot
Substratplättchen + $SiO_2$ + $TiO_2$ + Berliner Blau
Substratplättchen + $SiO_2$ + $SnO_2$ + $TiO_2$ + Berliner Blau
Substratplättchen + $SiO_2$ + $TiO_2$ / $Fe_2O_3$
Substratplättchen + $SiO_2$ + $SnO_2$ + $TiO_2$ / $Fe_2O_3$
Substratplättchen + $SiO_2$ + $TiO_2$ + $SiO_2$ + $TiO_2$
Substratplättchen + $SiO_2$ + $SnO_2$ + $TiO_2$ + $SiO_2$ + $SnO_2$ + $TiO_2$
Substratplättchen + $SiO_2$ + $Fe_2O_3$
Substratplättchen + $SiO_2$ + $Fe_3O_4$
Substratplättchen + $SiO_2$ + $TiO_2$ + $SiO_2$ + $TiO_2$/$Fe_2O_3$
Substratplättchen + $SiO_2$ + $Sn0_2$ + $TiO_2$ + $SiO_2$ + $TiO_2$/$Fe_2O_3$
Substratplättchen + $SiO_2$ + $TiO_2$ + $SiO_2$ + $TiO_2$ + $TiO_2$/$Fe_2O_3$
Substratplättchen + $SiO_2$ + $SnO_2$ + $TiO_2$ + $SiO_2$ + $TiO_2$ + $TiO_2$/$Fe_2O_3$
Substratplättchen + $SiO_2$ + $SnO_2$ + $TiO_2$ + $SiO_2$ + $SnO_2$ + $TiO_2$ + $TiO_2$/$Fe_2O_3$
Substratplättchen + $SiO_2$ + $TiO_2$/$Fe_2O_3$ + $SiO_2$ + $TiO_2$ + $TiO_2$/$Fe_2O_3$
Substratplättchen + $SiO_2$ + $TiO_2$/$Fe_2O_3$ + $SiO_2$ + $SnO_2$ + $TiO_2$ + $TiO_2$/$Fe_2O_3$
Substratplättchen + $SiO_2$ + $TiO_2$/$Fe_2O_3$ + $SiO_2$ + $TiO_2$
Substratplättchen + $SiO_2$ + $TiO_2$/$Fe_2O_3$ + $SiO_2$ + $SnO_2$ + $TiO_2$
Substratplättchen + $SiO_2$ + $TiO_2$/$Fe_2O_3$ + $SiO_2$ + $TiO_2$/$Fe_2O_3$
Substratplättchen + $SiO_2$ + $TiO_2$ + $SiO_2$ + $TiO_2$/$Fe_2O_3$
Substratplättchen + $SiO_2$ + $TiO_2$ + $SiO_2$ + $Fe_3O_4$
Substratplättchen + $SiO_2$ + $TiO_2$ + $SiO_2$ + $Cr_2O_3$
Substratplättchen + $SiO_2$ + $Cr_2O_3$
Substratplättchen + $SiO_2$ + Ag
Substratplättchen + $SiO_2$ + Au
Substratplättchen + $SiO_2$ + $Fe_2O_3$ + $SiO_2$ + $TiO_2$/$Fe_2O_3$
Substratplättchen + $TiO_2$ (Anatas) + $SiO_2$
Substratplättchen + $SnO_2$ + $TiO_2$ (Rutil) + $SiO_2$.

Die Belegung der rundlichen Substratplättchen mit einem Kreisformfaktor von 1,2-2 erfolgt vorzugsweise nasschemisch. Hierzu werden vorzugsweise die zur Herstellung von Perlglanzpigmenten entwickelten nasschemischen Beschichtungsverfahren angewendet. Derartige Verfahren sind z. B. beschrieben in DE 14 67 468, DE 19 59 988, DE 20 09 566, DE 22 14 545, DE 22 15 191, DE 22 44 298, DE 23 13 331, DE 25 22 572, DE 31 37 808, DE 31 37 809, DE 31 51 343, DE 31 51 354, DE 31 51 355, DE 32 11 602, DE 32 35 017 oder auch in weiteren dem Fachmann bekannten Patentdokumenten und sonstigen Publikationen.

Die Herstellung der erfindungsgemäßen Effektpigmente erfolgt in der Regel, indem die rundlichen Substratplättchen in Wasser suspendiert und mit ein oder mehreren hydrolysierbaren Metallsalzen bei einem für die Fällung geeigneten pH-Wert versetzt werden, der so gewählt wird, dass das Metalloxid bzw. Metalloxidhydrat direkt auf den Plättchen abgeschieden wird, ohne dass es zu wesentlichen Nebenfällungen kommt. Der pH-Wert wird üblicherweise durch gleichzeitiges Zudosieren einer Base oder Säure konstant gehalten. Die beschichteten Substrate werden nach dem Filtrieren und Waschen zunächst 20 - 60 min bei Temperaturen von 50 - 150 °C, vorzugsweise bei 80 - 120 °C, getrocknet und nachfolgend bei 600 bis 1200 °C, vorzugsweise bei 700 - 1000 °C, insbesondere bei 700 - 900 °C, für 0,3 - 1 h, vorzugsweise 0,5 - 0,8 h geglüht.

Weiterhin kann die Beschichtung, z.B. mit einer $TiO_2$-Schicht, auch in einem Wirbelbettreaktor durch Gasphasenbeschichtung erfolgen, wobei z. B. die in EP 0 045 851 A1 und EP 0 106 235 A1 zur Herstellung von Perlglanzpigmenten vorgeschlagenen Verfahren entsprechend angewendet werden können.

Die Belegung der Substratplättchen mit Titandioxid erfolgt vorzugsweise nasschemisch nach dem Chlorid- oder Sulfatprozess.

Zur zusätzlichen Erhöhung der Licht-, Wasser- und Wetterstabilität empfiehlt es sich häufig, in Abhängigkeit vom Einsatzgebiet, das fertige Effektpigment einer Nachbeschichtung oder Nachbehandlung zu unterziehen. Als Nachbeschichtungen bzw. Nachbehandlungen kommen beispielsweise die in den DE-PS 22 15 191, DE-OS 31 51 354, DE-OS 32 35 017 oder DE-OS 33 34 598 beschriebenen Verfahren in Frage. Durch diese Nachbeschichtung wird die chemische Stabilität weiter erhöht oder die Handhabung des Pigments, insbesondere die Einarbeitung in unterschiedliche Medien, erleichtert. Zur Verbesserung der Benetzbarkeit, Dispergierbarkeit und/oder Verträglichkeit mit den Anwendungsmedien können funktionelle Beschichtungen aus $Al_2O_3$ oder $ZrO_2$ oder deren Gemische bzw. Mischphasen auf die Pigmentoberfläche aufgebracht werden. Weiterhin sind organische, bzw. organisch/anorganisch kombinierte Nachbeschichtungen möglich, z.B. mit Silanen, wie beispielsweise beschrieben in der EP 0090259, EP 0 634 459, WO 99/57204, WO 96/32446, WO 99/57204, U.S. 5,759,255, U.S. 5,571,851, WO 01/92425, WO 2006/021386 A1 oder in J.J. Ponjeé, Philips Technical Review, Vol. 44, No. 3, 81 ff. und P.H. Harding J.C. Berg, J. Adhesion Sci. Technol. Vol. 11 No. 4, S. 471-493.

Bevorzugt besteht die äußere optionale Schutzschicht aus einer oder zwei Metalloxidschichten der Elemente Si, Al oder Ce. Insbesondere bevorzugt ist hierbei eine Schichtenfolge bei der zunächst eine Ceroxidschicht aufgebracht ist, der dann eine $SiO_2$-Schicht folgt, wie beispielsweise in der WO 2006/021386 A1 beschrieben.

Die äußere Schutzschicht kann des Weiteren an der Oberfläche organisch-chemisch modifiziert sein. Beispielsweise können ein oder mehrere Silane auf dieser äußeren Schutzschicht aufgebracht sein. Bei den Silanen kann es sich um Alkylsilane mit verzweigten oder unverzweigten Alkylresten mit 1 bis 24 C-Atomen, vorzugsweise 6 bis 18 C-Atomen handeln.

Bei den Silanen kann es sich aber auch um organofunktionelle Silane handeln, die eine chemische Anbindung an einen Kunststoff, ein Bindemittel eines Lackes oder einer Farbe, etc. ermöglichen.

Die vorzugsweise als Oberflächenmodifizierungsmittel verwendeten organofunktionellen Silane, die geeignete funktionelle Gruppen aufweisen, sind kommerziell verfügbar und werden beispielsweise von der Fa. Degussa, Rheinfelden, Deutschland, hergestellt und unter dem Handelsnamen "Dynasylan®" vertrieben. Weitere Produkte können von der Fa. OSi Specialties (Silquest®-Silane) oder von der Fa. Wacker, beispielsweise Standard- und $\alpha$-Silane aus der GENIOSIL®-Produktgruppe, bezogen werden.

Beispiele hierfür sind 3-Methacryloxypropyltrimethoxysilan (Dynasylan MEMO, Silquest A-174NT), Vinyltri(m)ethoxysilan (Dynasylan VTMO bzw. VTEO, Silquest A-151 bzw. A-171), 3-Mercaptopropyltri(m)ethoxysilan (Dynasylan MTMO oder 3201; Silquest A-189), 3-Glycidoxypropyltrimethoxysilan (Dynasylan GLYMO, Silquest A-187), tris-(3-Trimethoxysilylpropyl)isocyanurat (Silquest Y-11597), gamma-Mercaptopropyltrimethoxysilan (Silquest A-189), Bis-(3-Triethoxysilylpropyl)polysulfid (Silquest A-1289), Bis-(3-Triethoxysilyl)disulfid (Silquest A-1589), beta-(3,4-Epoxycyclohexyl)ethyltrimethoxysilan (Silquest A-186), Bis(triethoxysilyl)ethan (Silquest Y-9805), gamma-Isocyanatopropyltrimethoxysilan (Silquest A-Link 35, GENIOSIL GF40), (Methacryloxymethyl)tri(m)ethoxysilan (GENIOSIL XL 33, XL 36), (Methacryloxymethyl)(m)ethyldimethoxysilan (GENIOSIL XL 32, XL 34), Isocyanatomethyl)trimethoxysilan (GENIOSIL XL 43), (Isocyanatomethyl)methyldimethoxysilan (GENIOSIL XL 42), (Isocyanatomethyl)trimethoxysilan (GENIOSIL XL 43) 3-(Triethoxysilyl)propylbernsteinsäureanhydrid (GENIOSIL GF 20), (Methacryloxymethyl)methyldiethoxysilan, 2-Acryloxyethylmethyldimethoxysilan, 2-Methacryloxyethyltrimethoxysilan, 3-Acryloxypropylmethyldimethoxysilan, 2-Acryloxyethyltrimethoxysilan, 2-Methacryloxyethyltriethoxysilan, 3-Acryloxypropyltrimethoxysilan, 3-Acryloxypropyltripropoxysilan, 3-Methacryloxypropyltriethoxysilan, 3-Methacryloxypropyltriacetoxysilan, 3-Methacryloxypropylmethytdimethoxysilan, Vinyltrichlorsilan, Vinyltrimethoxysilan (GENIOSIL XL 10), Vinyltris(2-methoxyethoxy)silan (GENIOSIL GF 58), Vinyltriacetoxysilan.

Es ist aber auch möglich, andere organofunktionelle Silane auf den erfindungsgemäßen Effektpigmenten zu verwenden.

Weiterhin lassen sich, beispielsweise kommerziell von Degussa erhältliche, wässrige Vorhydrolysate einsetzen. Hierzu gehören u.a. wässriges, alkoholfreies Aminosilanhydrolysat (Dynasylan Hydrosil 1151), wässriges, alkoholfreies amino/alkylfunktionelles Siloxancooligomer (Dynasylan Hydrosil 2627), wässriges, alkoholfreies diaminolalkylfunktionelles Siloxancooligomer (Dynasylan Hydrosil 2776), wässriges, alkoholfreies amino/vinylfunktionelles Siloxancooligomer (Dynasylan Hydrosil 2907), wässriges, alkoholfreies amino/alkylfunktionelles Siloxancooligomer (Dynasylan Hydrosil 2909), wässriges, alkoholfreies epoxyfunktionelles Siloxanoligomer (Dynasylan Hydrosil 2926) oder wässriges, alkoholfreies amino/methacrylatfunktionelles Siloxancooligomer (Dynasylan Hydrosil 2929), oligomeres Diaminosilansystem (Dynasylan 1146), vinyl/alkylfunktionelles Siloxancooligomer (Dynasylan 6598), vinyl- und methoxygruppenhaltiges Vinylsilankonzentrat (oligomeres Siloxan) (Dynasylan 6490) oder oligomeres kurzkettiges alkylfunktionelles Silan (Dynasylan 9896).

Bei einer bevorzugten Ausführungsform enthält das organofunktionelle Silangemisch neben wenigstens einem Silan ohne funktionelle Bindungsgruppe wenigstens ein aminofunktionelles Silan. Die Aminofunktion ist eine funktionelle Gruppe, die mit den meisten in Bindemitteln vorhandenen Gruppen eine oder mehrere chemische Wechselwirkungen eingehen kann. Dies kann eine kovalente Bindung, wie z.B. mit Isocyanat- oder Carboxylatfunktionen des Bindemittels, oder Wasserstoffbrückenbindungen wie mit OH- oder COOR-Funktionen oder auch ionische Wechselwirkungen beinhalten. Eine Aminofunktion ist daher für den Zweck der chemischen Anbindung des Effektpigmentes an verschiedenartige Bindemittel sehr gut geeignet.

Bevorzugt werden hierzu folgende Verbindungen genommen: Aminopropyltrimethoxysilan (Dynasylan AMMO; Silquest A-1110), Aminopropyltriethoxysilan (Dynasylan AMEO) oder N-(2-Aminoethyl)-3-aminopropyltrimethoxysilan (Dynasylan DAMO, Silquest A-1120) oder N-(2-Aminoethyl)-3-aminopropyltriethoxysilan, Triamino-funktionelles Trimethoxysilan (Silquest A-1130), bis-(gamma-Trimethoxysilylpropyl)amin (Silquest A-1170), N-ethyl-gammaaminoisobutyltrimethoxysilan (Silquest A-Link 15), N-Phenylgammaaminopropyltrimethoxysilan (Silquest Y-9669), 4-Amino-3,3-dimethylbutyltrimethoxysilan (Silquest Y-11637), N-Cyclohexylaminomethylmethyldiethoxysilan (GENIOSIL XL 924), (N-Cyclohexylaminomethyl)triethoxysilan (GENIOSIL XL 926), (N-Phenylaminomethyl)trimethoxysilan (GENIOSIL XL 973) und deren Mischungen.

Bei einer weiterhin bevorzugten Ausführungsform ist das Silan ohne funktionelle Bindungsgruppe ein Alkylsilan. Das Alkylsilan weist vorzugsweise die Formel

$$R_{(4-z)}Si(X)_z$$

auf.

Hierbei ist z eine ganze Zahl von 1 bis 3, R ist eine substituierte oder unsubstituierte, unverzweigte oder verzweigte Alkylkette mit 10 bis 22 C-Atomen und X steht für eine Halogen- und/oder Alkoxygruppe. Bevorzugt sind Alkylsilane mit Alkylketten mit mindestens 12 C-Atomen. R kann auch zyklisch mit Si verbunden sein, wobei in diesem Fall z üblicherweise 2 ist.

Ein derartiges Silan bewirkt eine stärkere Hydrophobierung der Pigmentoberfläche. Diese wiederum führt dazu, dass das derart beschichtete Effektpigment in der Lackbeschichtung tendenziell nach oben aufschwimmt. Bei plättchenförmigen Effektpigmenten wird ein derartiges Verhalten als "leafing"-Verhalten bezeichnet.

Eine Silanmischung bestehend aus mindestens einem Silan, welches wenigstens eine funktionelle Gruppe besitzt, die eine Anbindung an das Bindemittel ermöglicht, und einem, in Wasser unlöslichen oder kaum löslichen Alkylsilan ohne Aminogruppe, ermöglicht optimale anwendungstechnische Eigenschaften der Effektpigmente. Eine solche organisch-chemische Oberflächenmodifizierung führt dazu, dass sich die Effektpigmente in einer Lack- oder Farbschicht ausgezeichnet orientieren, d.h. im Wesentlichen planparallel zu dem lackierten bzw. angestrichenen Untergrund, und zugleich chemisch mit dem Bindemittelsystem des Lackes bzw. der Farbe reagieren und mithin kovalent in der Lack- bzw. Farbschicht gebunden sind. Derartige Lack- bzw. Farbschichten weisen eine erhöhte mechanische und chemische Beständigkeit gegenüber Umwelteinflüssen, wie Wetter etc., auf.

Die erfindungsgemäßen Effektpigmente zeichnen sich neben der hohen Farbsättigung und einem sehr hellen und hohen Glanz durch eine sehr hohe Deckkraft und geringe Kantenrauhigkeit aus. Die Pigmente sind daher auch sehr gut aufgrund ihres guten Skin-Feelings für kosmetische Formulierungen geeignet.

Da die erfindungsgemäßen Effektpigmente neben einer hohen Deckkraft, einen hellen und starken Glanz mit hoher Farbsättigung aufweisen, lassen sich mit ihnen besonders wirksame Effekte in den verschiedenen Anwendungsmedien erzielen.

Es versteht sich von selbst, dass für die verschiedenen Anwendungszwecke die erfindungsgemäßen Effektpigmente auch vorteilhaft in Abmischung mit organischen Farbstoffen, organischen Pigmenten oder anorganischen Pigmenten, wie z. B. transparenten und deckenden Weiß-, Bunt- und Schwarzpigmenten sowie mit plättchenförmigen Eisenoxiden, holographischen Pigmenten, LCPs (Liquid Crystal Polymers) oder mit Perlglanzpigmenten, etc., verwendet werden können. Dabei sind den Mischungsverhältnissen und Konzentrationen keine Grenzen gesetzt.

Die erfindungsgemäßen Effektpigmente können in jedem Gewichtsverhältnis mit handelsüblichen Pigmenten und Füllstoffen gemischt werden. Vorzugsweise beträgt das Verhältnis 1 : 1 bis 9 : 1. Sofern die erfindungsgemäßen Effektpigmente mit Füllstoffen gemischt werden, kann das Mischungsverhältnis auch 99 :1 bis 1 : 99 betragen.

Die erfindungsgemäßen Effektpigmente sind mit einer Vielzahl von Farbsystemen kompatibel, vorzugsweise aus dem Bereich der Lacke, Farben und Druckfarben. Für die Herstellung der Druckfarben für z. B. den Tiefdruck, Flexodruck, Offsetdruck, Offsetüberdruck-Lackierung ist eine Vielzahl von Bindern, insbesondere wasserlösliche Typen, geeignet, wie sie z. B. von den Firmen BASF, Marabu, Pröll, Sericol, Hartmann, Gebr. Schmidt, Sicpa, Aarberg, Siegberg, GSB-Wahl, Follmann, Ruco oder Coates Screen INKS GmbH vertrieben werden. Die Druckfarben können auf Wasserbasis oder Lösemittelbasis aufgebaut sein. Weiterhin sind die erfindungsgemäßen Effektpigmente auch für die Lasermarkierung von Papier und Kunststoffen, sowie für Anwendungen im Agrarbereich, z. B. für Gewächshausfolien, sowie z. B. für die Farbgebung von Zeltplanen, geeignet.

Die erfindungsgemäßen Effektpigmente können zur Pigmentierung von Lacken, Druckfarben, Kunststoffen, Agrarfolien, Saatgutbeschichtung, Lebensmitteleinfärbungen, Knopfpasten, Arzneimittelüberzügen oder kosmetischen Formulierungen, wie Lippenstifte, Nagellacke, Presspuder, Shampoos, Seifen, lose Puder und Gele, verwendet werden.

EP 2 607 432 A1

Die Konzentration des Pigments im zu pigmentierenden Anwendungssystem liegt in der Regel zwischen 0,1 und 70 Gew.%, vorzugsweise zwischen 0,1 und 50 Gew.% und insbesondere zwischen 0,5 und 10 Gew.%, bezogen auf den Gesamtfestkörpergehalt des Systems. Sie ist in der Regel abhängig vom konkreten Anwendungsfall.

In Kunststoffen enthaltend die erfindungsgemäßen Effektpigmente, vorzugsweise in Mengen von 0,01 bis 50 Gew.%, insbesondere 0,1 bis 7 Gew.%, lassen sich besonders ausgeprägte Farbeffekte erzielen.

Im Lackbereich, insbesondere im Automobillack, werden die Effektpigmente, auch für 3-Schichtaufbauten in Mengen von 0,1-20 Gew.%, vorzugsweise 1 bis 10 Gew.%, eingesetzt.

Im Lack haben die erfindungsgemäßen Effektpigmente den Vorteil, dass der angestrebte Glanz durch eine einschichtige Lackierung (Einschichtsystem bzw. Base coat im 2-Schichtaufbau) erzielt wird. Im Vergleich mit Lackierungen, die beispielsweise ein Mehrschichtpigment auf Basis von Glimmer bzw. ein herkömmliches, auf einem Substrat mit breiter Dickenverteilung basierendes Perlglanzpigment, statt der erfindungsgemäßen Effektpigmente enthalten, zeigen Lackierungen mit den erfindungsgemäßen Pigmenten eine deutlichere Tiefenwirkung und einen stärker ausgeprägten Farb- und Glanzeffekt.

Die erfindungsgemäßen Effektpigmente können auch vorteilhaft in der dekorativen und pflegenden Kosmetik eingesetzt werden. Die Einsatzkonzentration reicht von 0,01 Gew.% im Shampoo bis zu 100 Gew. % bei losen Pudern. Bei einer Mischung der erfindungsgemäßen Pigmente mit Füllstoffen, vorzugsweise mit sphärischen Füllstoffen, wie z. B. $SiO_2$, kann die Konzentration bei 0,01-70 Gew.% in der Formulierung liegen. Die kosmetischen Produkte, wie z.B. Nagellacke, Presspuder, Shampoos, lose Puder und Gele, zeichnen sich durch besonders interessante Farbeffekte und einen hohen Glanz aus.

Weiterhin können die erfindungsgemäßen Effektpigmente in Badezusätzen, Zahnpasten und zur Veredlung von Lebensmitteln, z. B. Masse-Einfärbung und/oder Überzüge von Bonbons, Weingummi, wie z.B. Gummibärchen, Pralinen, Lakritze, Konfekt, Zuckerstangen, Puddings, Brausegetränke, Limonaden, etc., oder als Überzug, z.B. bei Dragees und Tabletten im Pharmabereich, eingesetzt werden.

Die erfindungsgemäßen Effektpigmente können weiterhin mit handelsüblichen Füllern gemischt werden. Als Füllstoffe sind z.B. zu nennen natürlicher und synthetischer Glimmer, Nylon Powder, reine oder gefüllte Melaminharze, Talkum, Gläser, Kaolin, Oxide oder Hydroxide von Aluminium, Magnesium, Calcium, Zink, BiOCl, Bariumsulfat, Calciumsulfat, Calciumcarbonat, Magnesiumcarbonat, Kohlenstoff, sowie physikalische oder chemische Kombinationen dieser Stoffe. Bezüglich der Partikelform des Füllstoffes gibt es keine Einschränkungen. Sie kann den Anforderungen gemäß z. B. plättchenförmig, sphärisch oder nadelförmig sein.

Selbstverständlich können die erfindungsgemäßen Effektpigmente in den Formulierungen auch mit jeder Art von kosmetischen Roh- und Hilfsstoffen kombiniert werden. Dazu gehören u.a. Öle, Fette, Wachse, Filmbildner, Konservierungsmittel und allgemein anwendungstechnische Eigenschaften bestimmende Hilfsstoffe, wie z. B. Verdicker und rheologische Zusatzstoffe, wie z.B. Bentonite, Hektorite, Siliziumdioxide, Ca-Silicate, Gelatinen, hochmolekulare Kohlenhydrate und/oder oberflächenaktive Hilfsmittel etc.

Die die erfindungsgemäßen Effektpigmente enthaltenden Formulierungen können dem lipophilen, hydrophilen oder hydrophoben Typ angehören. Bei heterogenen Formulierungen mit diskreten wässrigen und nichtwässrigen Phasen können die erfindungsgemäßen Effektpigmente in jeweils nur einer der beiden Phasen enthalten oder auch über beide Phasen verteilt sein.

Die pH-Werte der Formulierungen können zwischen 1 und 14, bevorzugt zwischen 2 und 11 und besonders bevorzugt zwischen 5 und 8 liegen.

Den Konzentrationen der erfindungsgemäßen Effektpigmente in der Formulierung sind keine Grenzen gesetzt. Sie können - je nach Anwendungsfall -zwischen 0,001 (rinse-off-Produkte, z.B. Duschgele) und 100 % (z.B. Glanzeffekt-Artikel für besondere Anwendungen) liegen.

Die erfindungsgemäßen Effektpigmente können weiterhin auch mit kosmetischen Wirkstoffen kombiniert werden. Geeignete Wirkstoffe sind z.B. Insect Repellents, UV A/BC-Schutzfilter (z.B. OMC, B3, MBC), Anti-Ageing-Wirkstoffe, Vitamine und deren Derivate (z.B. Vitamin A, C, E etc.), Selbstbräuner (z.B. DHA, Erytrolyse, u.a.) sowie weitere kosmetische Wirkstoffe wie z.B. Bisabolol, LPO, Ectoin, Emblica, Allantoin, Bioflavanoide und deren Derivate.

Bei der Pigmentierung von Bindemittelsystemen z. B. für Farben und Druckfarben für den Tiefdruck, Offsetdruck oder Siebdruck, oder als Vorprodukt für Druckfarben, hat sich der Einsatz der erfindungsgemäßen Effektpigmente in Form von hochpigmentierten Pasten, Granulaten, Pellets, etc., als besonders geeignet erwiesen. Die Effektpigmente werden in der Regel in die Druckfarbe in Mengen von 2-35 Gew.%, vorzugsweise 5-25 Gew.%, und insbesondere 8-20 Gew.% eingearbeitet. Offsetdruckfarben können die Pigmente bis zu 40 Gew.% und mehr enthalten. Die Vorprodukte für die Druckfarben, z.B. in Granulatform, als Pellets, Briketts, etc., enthalten neben dem Bindemittel und Additiven bis zu 98 Gew.% der erfindungsgemäßen Pigmente. Die Druckfarben enthaltend die erfindungsgemäßen Pigmente zeigen reinere Farbtöne als mit herkömmlichen Effektpigmenten. Die Partikeldicken der erfindungsgemäßen Effektpigmente sind relativ gering und bedingen daher eine besonders gute Verdruckbarkeit.

Die erfindungsgemäßen Effektpigmente sind weiterhin geeignet zur Herstellung von fließfähigen Pigmentpräparationen und Trockenpräparaten, insbesondere für Druckfarben, enthaltend ein oder mehrere erfindungsgemäße Pigmente, Bindemittel und optional ein oder mehrere Additive.

Gegenstand der vorliegenden Erfindung ist weiterhin die Verwendung der erfindungsgemäßen Effektpigmente in Farben, Lacken, Pulverlacken, Autolacken und Industrielacken, Druckfarben, Kunststoffen, Knopfpasten, keramischen Materialien, Gläsern, zur Saatgutbeschichtung, als Absorber bei der Lasermarkierung von Kunststoffen, Gläsern, Pappen und Papieren, als Absorber beim Laserschweißen von Kunststoffen, zur Farbgebung von Lebensmittel- und Pharmaprodukten, zur Farbgebung von Überzügen (Coatings) von Lebensmittel- und Pharmaprodukten, in kosmetischen Formulierungen und in fälschungssicheren Wertdokumenten, wie z.B. Geldscheinen, Kreditkarten, Ausweispapiere, etc. Weiterhin sind die erfindungsgemäßen Pigmente auch zur Herstellung von Pigmentpräparationen sowie zur Herstellung von Trockenpräparaten, wie z. B. Granulaten, Chips, Pellets, Briketts, etc., geeignet. Die Trockenpräparate sind insbesondere für Druckfarben und für kosmetische Formulierungen geeignet.

Gegenstand der Erfindung sind somit auch Formulierungen enthaltend die erfindungsgemäßen Effektpigmente.

Gegenstand der Erfindung sind insbesondere Formulierungen, die neben den erfindungsgemäßen Effektpigmenten mindestens einen Bestandteil ausgewählt aus der Gruppe der Absorptionsmittel, Adstringenzien, antimikrobiellen Stoffen, Antioxidantien, Antiperspirantien, Antischaummitteln, Antischuppenwirkstoffen, Antistatika, Bindemitteln, biologischen Zusatzstoffen, Bleichmitteln, Chelatbildnern, Desodorierungsmitteln, Emollentien, Emulgatoren, Emulsionsstabilisatoren, Farbstoffen, Feuchthaltemitteln, Filmbildnern, Füllstoffen, Geruchsstoffen, Geschmacksstoffen, Insect Repellents, Konservierungsmitteln, Korrosionsschutzmitteln, kosmetischen Ölen, Lösungsmitteln, Oxidationsmitteln, pflanzlichen Bestandteilen, Puffersubstanzen, Reduktionsmitteln, Tensiden, Treibgasen, Trübungsmitteln, UV-Filtern und UV-Absorbern, Vergällungsmitteln, Viskositätsreglern, Parfüm und Vitaminen enthalten.

Die nachfolgenden Beispiele sollen die Erfindung erläutern, ohne sie jedoch zu begrenzen.

**Beispiele**

I. Herstellung der Substrate

Beispiel 1 a

1 kg Muskovit-Glimmer, der in Form grober Flocken vorliegt, wird durch einen Kollerprozess auf eine Teilchengröße von unter 200 $\mu$m gemahlen. Die so entstehenden feinen Glimmerplättchen werden in eine Rotor-Stator-Mühle gegeben und mit 1 l VE-Wasser versetzt. Die entstehende Suspension wird 5 Stunden in der Mühle vermahlen, wobei gleichzeitig die Oberfläche der Plättchen glatt poliert wird. Die mechanische Belastung der Teilchen während des Mahlschritts wird dabei so gewählt, dass eine permanente Scherung zur weiteren schonenden Delaminierung von Teilchen und zur Glättung der Kanten und Oberflächen führt. Eine enge Teilchengrößenverteilung wird durch einen anschließenden Klassierschritt in Form von mehreren (mindestens 3 Stufen) Sedimentationsstufen erreicht. Die so erzeugten Glimmerplättchen weisen eine Teilchengrößenverteilung von 10 - 40 $\mu$m, eine Dickenverteilung von 0,2 bis 0,6 $\mu$m (jeweils 90 % aller Teilchen) sowie eine glatt polierte Oberfläche mit nur wenigen scharfen Kanten auf. Der Kreisformfaktor beträgt 1,6.

Beispiel 1 b

Analog Beispiel 1 a werden 1 kg synthetische Glimmerflocken behandelt. Man erhält synthetische Glimmerplättchen mit einem Kreisformfaktor von 1,6.

II. Belegung der Substratplättchen

Beispiel 2.1: Natürlicher Glimmer + $SnO_2$ + $TiO_2$

100 g natürliche Glimmerplättchen aus Beispiel 1 a werden in 2 l VE-Wasser unter Rühren auf 75 °C erwärmt. Durch Zutropfen einer $SnCl_4$-Lösung (22 g/l) wird der Belegungs-pH-Wert von 1,8 eingestellt. Anschließend wird der Rest von 100 ml $SnCl_4$-Lösung (22 g/l) zudosiert. Dabei wird der pH-Wert mit 32 %iger Natronlauge konstant auf 1,8 gehalten. Nach beendeter Zugabe wird 10 Minuten nachgerührt.

Bei konstantem pH-Wert werden nun 490 ml einer $TiOCl_2$-Lösung (400 g $TiCl_4$/l) bis zum Erreichen des Farbendpunktes (gelb) zudosiert, wobei der pH-Wert durch gleichzeitiges Zutropfen von 32 %iger Natronlauge konstant bei 1,8 gehalten wird.

Nach beendeter Zugabe wird 10 Minuten nachgerührt, die Suspension abgesaugt und mit VE-Wasser salzfrei gewaschen. Nach Trocknung bei 120 °C (24 h) wird das Pigment 45 Minuten bei 800 °C kalziniert.

Man erhält ein hochglänzendes, farbstarkes Pigment mit gelber Interferenzfarbe.

Beispiel 2.2: Synthetischer Glimmer + SnO$_2$ + TiO$_2$ + SiO$_2$ + SnO$_2$ + TiO$_2$

75 g synthetische Glimmerplättchen aus Beispiel 1 b werden mit 1500 ml VE-Wasser unter Rühren auf 75 °C erwärmt. Nun wird mit Salzsäure (15 % HCl) der pH-Wert der Suspension auf 1,8 eingestellt. Anschließend werden 75 ml SnCl$_4$-Lösung (22 g/l) zudosiert. Dabei wird der pH-Wert mit 32 %iger Natronlauge konstant auf 1,8 gehalten. Nach beendeter Zugabe wird 10 Minuten nachgerührt.

Es folgt die Zudosierung einer TiOCl$_2$-Lösung (400 g TiCl$_4$/l) bis zum Farbendpunkt (gelb), wobei der pH-Wert durch gleichzeitiges Zutropfen von 32 %iger Natronlauge konstant bei 1,8 gehalten wird.

Durch Farbmessung während des Belegungsprozesses wird die Koloristik während der Herstellung des Pigmentes kontrolliert und der Fällungsprozess nach dem Buntton (Bunttonwinkel arc tan b*/a*) gesteuert.

Die Belegung wird gestoppt und anschließend wird 15 Minuten nachgerührt.

Der pH-Wert wird anschließend mit verdünnter Natronlauge auf pH=9,0 eingestellt. Dann werden 100 ml einer Natronwasserglaslösung mit einem Gehalt von 2 % zugegeben, wobei der pH-Wert mittels Salzsäure (10 % HCl) konstant gehalten wird. Anschließend wird 30 Minuten nachgerührt.

Durch Zutropfen von Salzsäure (w = 20 %) wird der pH-Wert auf 1,8 abgesenkt. 75 ml SnCl$_4$-Lösung (22 g/l) werden zudosiert. Dabei wird der pH-Wert mit 32 %iger Natronlauge konstant auf 1,8 gehalten. Nach beendeter Zugabe wird 10 Minuten nachgerührt. Bei konstantem pH-Wert werden nun noch 300 ml TiOCl$_2$-Lösung (400 g TiCl$_4$/l) bis zum Erreichen des Farbendpunktes (grün) zudosiert, wobei der pH-Wert durch gleichzeitiges Zutropfen von 32 %iger Natronlauge konstant bei 1,8 gehalten wird.

Nach einer Nachrührzeit von 15 Minuten wird das Pigment durch Filtration von der überstehenden Reaktionslösung abgetrennt und salzfrei gewaschen. Nach einer Trocknung bei 120 °C (24 h) wird das Pigment 45 Minuten bei 800 °C kalziniert.

Man erhält ein hochglänzendes, farbstarkes Pigment mit grüner Interferenzfarbe.

III. Vergleich der Lab-Werte von Iriodin® 205 mit dem Pigment aus Beispiel 2.1

Die Lab-Werte (gemessen mit Eta-Messgerät, Messwinkel 75°/95°) werden anhand von Schwarz-Weiß-Lackkarten (NC-Lack) für Iriodin® 205 (gelbes Interferenzpigment auf Glimmerplättchen) der Fa. Merck KGaA und für das Pigment aus Beispiel 2.1 bestimmt:

|  | Iriodin® 205 Stand der Technik | Pigment aus Beispiel 2.1 Erfindung |
|---|---|---|
| L-Wert | 119,8 | 126,6 |
| a-Wert | 8,7 | 10,3 |
| b-Wert | 63,0 | 74,6 |
| Chroma | 63,6 | 75,3 |

$$\sqrt{(a^2 + b^2)}$$

Durch die deutlich erhöhten a- und b-Werte des erfindungsgemäßen Pigments wird eine signifikant verbesserte Chroma erreicht, während der hohe L-Wert auf einen erhöhten Glanz hinweist.

**Anwendungsbeispiele**

Beispiel A: Duschgel

Phase A

| Rohstoff | Bezugsquelle | INCI | [%] |
|---|---|---|---|
| Pigment aus Beispiel 2.1 | Merck KGaA | | 0,10 |
| Keltrol T | Kelco | Xanthan Gum | 0,75 |
| Wasser, demineralisiert | | Aqua (Water) | 64,95 |

Phase B

| Rohstoff | Bezugsquelle | INCI | [%] |
|---|---|---|---|
| Plantacare 2000 UP | Cognis GmbH | Decyl Glucoside | 20,00 |
| Texapon ASV 50 | Cognis GmbH | Sodium Laureth Sulfate, Sodium Laureth-8 Sulfate, Magnesium Laureth Sulfate, Magnesium Laureth-8 Sulfate, Sodium Oleth Sulfate, Magnesium Oleth Sulfate | 3,60 |
| Bronidox L | Cognis GmbH | Propylene Glycol, 5-Bromo-5-Nitro-1,3-Dioxane | 0,20 |
| Parfümöl Everest 79658 SB | Haarmann & Reimer GmbH | Parfum | 0,05 |
| 1 % FD&C Blue No. 1 in Wasser | BASF AG | Aqua (Water), CI 42090 (FD&C Blue No. 1) | 0,20 |

Phase C

| Rohstoff | Bezugsquelle | INCI | [%] |
|---|---|---|---|
| Citronensäure Monohydrat | Merck KGaA/Rona® | Citric Acid | 0,15 |
| Wasser, demineralisiert | | Aqua (Water) | 10,00 |

Herstellung:

Für Phase A das Interferenzpigment in das Wasser einrühren. Keltril T unter Rühren langsam einstreuen und rühren bis es gelöst ist. Die Phasen B und C nacheinander hinzufügen und dabei langsam rühren bis alles homogen verteilt ist. pH-Wert auf 6,0 bis 6,4 einstellen.

Beispiel B: Nagellack

| Rohstoff | Bezugsquelle | INCI | [%] |
|---|---|---|---|
| Pigment aus Beispiel 2.2 | Merck KGaA | | 2,00 |
| Thixotrope Nagellack-Base 1348 | International Lacquers S.A. | Toluene, Ethyl Acetate, Butyl Acetate, Nitrocellulose, Tosylamide/Formaldehyde Resin, Dibutyl Phthalate, Isopropyl Alcohol, Stearalkonium Hectorite, Camphor, Acrylates Copolymer, Benzophenone-1 | 98,00 |

Herstellung:

Das Interferenzpigment wird zusammen mit der Lackbase eingewogen, gut mit einem Spatel von Hand vermischt und anschließend 10 min bei 1000 Upm gerührt.

Beispiel C: Lacksystem

90 Gew. % Hydroglasur BG/S farblos (Wasserlack der Fa. Ernst Diegel GmbH)
10 Gew. % Pigment aus Beispiel 2.2
Lackieren durch Aufsprühen bei 80 °C
5 min vortrocknen bei 80 °C
20 min einbrennen bei 180 °C

Beispiel D: Kunststoff

1 kg Polystyrolgranulat werden in einem Taumelmischer mit 5 g Haftmittel gleichmäßig benetzt. Dazu werden dann 42 g grünes Interferenzpigment aus Beispiel 2.2 zugegeben und 2 min lang gemischt. Dieses Granulat wird auf einer Spritzgießmaschine unter üblichen Bedingungen zu Stufenplättchen mit den Maßen 4 x 3 x 0,5 cm verarbeitet. Die Stufenplättchen zeichnen sich durch ihren ausgeprägten Sparkle-Effekt aus.

Beispiel E: Einfärbung von Süßwaren

Rohware: Brause-Bonbons weiß

Sprühlösung:

94 % alkoholische Schellacklösung der Fa. Kaul
6 % Effektpigment aus Beispiel 2.1

Die Brausebonbons werden mit einer Interferenzpigment/Schellacklösung besprüht bis der gewünschte Farbauftrag erreicht ist. Eine nachträgliche Trocknung mit Kaltluft ist möglich.

Beispiel F: Autolack

Das Pigment aus Beispiel 2.2 lässt sich leicht in Autolacke einarbeiten. Dazu wird die Lackbasis unter Rühren mit dem Pigment aus Beispiel 2.2 versetzt. Der Rührvorgang wird fortgesetzt bis sich das Pigment gleichmäßig im Lack verteilt hat. Der eingefärbte Lack wird auf schwarz und weiß beschichtete Aluminiumprüfbleche aufgesprüht.

Herstellung der Lackbleche:

| | |
|---|---|
| Lack : | Herberts Basislack 419982 |
| Pigmentierung: | 5 % |
| Trockenschichtdicke: | 15 $\mu$m |
| Spritzpistole: | Sprimag S 233; Düsendurchmesser: 1,5 mm |
| Spritzdruck: | 4 bar |
| Abstand Düse-Substrat: | 27 cm |

Beispiel G: Flexodruck

Herstellung der Druckfarbe:

Das Pigment aus Beispiel 2.1 wird mit prewetting Byk 348 (0,6%) vorbenetzt und in einer Konzentration von 22,9 % in das Bindemittel eingearbeitet.

Bindemittel: Koustom Kote 9000/ USA, wasserbasiert

Die Paste wird mit Wasser verdünnt bis eine Viskosität von 40 sec mit dem 4mm-Erichsen-Becher bei 25 °C erreicht ist.

Die Pigmente werden von einem Anilox-Keramik-Zylinder (24 ccm/m$^2$) über ein Lackplattenklischee auf matt-schwarzes Kunstdruckpapier gedruckt. Die erfindungsgemäßen Pigmente zeigen eine hohe Farbintensität.

Die Produkte der Anwendungsbeispiele A - G zeichnen sich durch ihren hohen Glanz, eine hohe Farbintensität und eine hohe Farbreinheit aus.

**Patentansprüche**

1. Effektpigment basierend auf einem plättchenförmigen Substrat, **dadurch gekennzeichnet, dass** das Substrat mit einem Kreisformfaktor (Umfang$^2$ / Fläche normiert auf einen Kreis) von 1,2 - 2 mit mindestens einer hochbrechenden Schicht mit einem Brechungsindex von n ≥ 1,8 beschichtet ist.

2. Effektpigment nach Anspruch 1, **dadurch gekennzeichnet, dass** das Substrat einen Kreisformfaktor von 1,2 - 1,8 hat.

3. Effektpigment nach Anspruch 1 oder 2, **dadurch gekennzeichnet, dass** das Substrat eine Partikelgröße von 5-60 μm aufweist.

4. Effektpigment nach einem oder mehreren der Ansprüche 1 bis 3, **dadurch gekennzeichnet, dass** das Substrat eine Dicke von 0,2-0,6 μm aufweist.

5. Effektpigment nach einem oder mehreren der Ansprüche 1 bis 4, **dadurch gekennzeichnet, dass** das plättchenförmige Substrat ausgewählt ist aus der Gruppe synthetisches Glimmerplättchen, natürliches Glimmerplättchen, $SiO_2$-Plättchen, $Al_2O_3$-Plättchen, Glasplättchen, Eisenoxidplättchen, Graphitplättchen, $TiO_2$-Plättchen oder Gemische davon.

6. Effektpigment nach einem oder mehreren der Ansprüche 1 bis 5, **dadurch gekennzeichnet, dass** das plättchenförmige Substrat ein Glimmer- oder Glasplättchen ist.

7. Effektpigment nach einem oder mehreren der Ansprüche 1 bis 6, **dadurch gekennzeichnet, dass** das plättchenförmige Substrat ein natürliches Glimmerplättchen ist.

8. Effektpigment nach einem oder mehreren der Ansprüche 1 bis 7, **dadurch gekennzeichnet, dass** das Substratplättchen mit mindestens einer hochbrechenden Schicht mit einem Brechungsindex von ≥ 1,8 beschichtet sind.

9. Effektpigment nach einem oder mehreren der Ansprüche 1 bis 8, **dadurch gekennzeichnet, dass** die hochbrechende Schicht ausgewählt ist aus der Gruppe der Metalloxide, Metallsulfide, Eisentitanate, Eisenoxidhydrate, Titansuboxide, Metalle, sowie Mischungen oder Mischphasen der genannten Verbindungen.

10. Effektpigment nach einem oder mehreren der Ansprüche 1 bis 9, **dadurch gekennzeichnet, dass** die hochbrechende Schicht aus $TiO_2$, $BiOCl$, $Ce_2O_3$, $Cr_2O_3$, $CoO$, $Co_3O_4$, $Fe_2O_3$, $Fe_3O_4$, $FeOOH$, $NiO$, $SnO_2$, $VO_2$, $V_2O_3$, $ZrO_2$, $ZnO$, $CoAl_2O_4$, $BiVO_4$, Eisentitanaten, Eisenoxidhydraten, $Ti_3O_5$, $Ti_2O_3$, $TiO$, $Ce_2S_3$, $MoS_2$, Aluminium, Chrom, Nickel, Silber, Gold, Titan, Kupfer oder deren Legierungen sowie Mischungen bzw. Mischphasen der genannten Verbindungen untereinander besteht.

11. Effektpigment nach einem oder mehreren der Ansprüche 1 bis 10, **dadurch gekennzeichnet, dass** die hochbrechende Schicht aus ein oder mehreren Metalloxiden besteht.

12. Effektpigment nach einem oder mehreren der Ansprüche 1 bis 11, **dadurch gekennzeichnet, dass** das Substrat mit mindestens einer hochbrechenden (n ≥ 1,8) und mindestens einer niedrigbrechenden Schicht (n < 1,8) beschichtet ist.

13. Effektpigment nach einem oder mehreren der Ansprüche 1 bis 12, **dadurch gekennzeichnet, dass** die niedrigbrechende Schicht ausgewählt ist aus der Gruppe $SiO_2$, $Al_2O_3$, $AlO(OH)$, $MgF_2$.

14. Effektpigment nach einem oder mehreren der Ansprüche 1 bis 13, **dadurch gekennzeichnet, dass** das Effektpigment folgende Schichtenabfolgen auf dem Substratplättchen aufweist:

    Substratplättchen + $TiO_2$ (Anatas)
    Substratplättchen + $SnO_2$ + $TiO_2$ (Rutil)
    Substratplättchen + $TiO_2$ + Erdalkalititanat
    Substratplättchen + $SnO_2$ + $TiO_2$ + Erdalkalititanat
    Substratplättchen + $TiO_2$ + $Fe_2O_3$
    Substratplättchen + $SnO_2$ + $TiO_2$ + $Fe_2O_3$

EP 2 607 432 A1

Substratplättchen + $TiO_2$ + $Fe_3O_4$
Substratplättchen + $SnO_2$ + $TiO_2$ + $Fe_3O_4$
Substratplättchen + $TiO_2$ + $Cr_2O_3$
Substratplättchen + $SnO_2$ + $TiO_2$ + $Cr_2O_3$
Substratplättchen + $TiO_2$ + Karminrot
Substratplättchen + $SnO_2$ + $TiO_2$ + Karminrot
Substratplättchen + $TiO_2$ + Berliner Blau
Substratplättchen + $SnO_2$ + $TiO_2$ + Berliner Blau
Substratplättchen + $TiO_2$ / $Fe_2O_3$
Substratplättchen + $SnO_2$ + $TiO_2$ / $Fe_2O_3$
Substratplättchen + $TiO_2$ + $SiO_2$ + $TiO_2$
Substratplättchen + $SnO_2$ + $TiO_2$ + $SiO_2$ + $SnO_2$ + $TiO_2$
Substratplättchen + $Fe_2O_3$
Substratplättchen + $Fe_3O_4$
Substratplättchen + $TiO_2$ + $SiO_2$ + $TiO_2$/$Fe_2O_3$
Substratplättchen + $SnO_2$ + $TiO_2$ + $SiO_2$ + $TiO_2$/$Fe_2O_3$
Substratplättchen + $TiO_2$ + $SiO_2$ + $TiO_2$ + $TiO_2$/$Fe_2O_3$
Substratplättchen + $SnO_2$ + $TiO_2$ + $SiO_2$ + $TiO_2$ + $TiO_2$/$Fe_2O_3$
Substratplättchen + $SnO_2$ + $TiO_2$ + $SiO_2$ + $SnO_2$ + $TiO_2$ + $TiO_2$/$Fe_2O_3$
Substratplättchen + $TiO_2$/$Fe_2O_3$ + $SiO_2$ + $TiO_2$ + $TiO_2$/$Fe_2O_3$
Substratplättchen + $TiO_2$/$Fe_2O_3$ + $SiO_2$ + $SnO_2$ + $TiO_2$ + $TiO_2$/$Fe_2O_3$
Substratplättchen + $TiO_2$/$Fe_2O_3$ + $SiO_2$ + $TiO_2$
Substratplättchen + $TiO_2$/$Fe_2O_3$ + $SiO_2$ + $SnO_2$ + $TiO_2$
Substratplättchen + $TiO_2$/$Fe_2O_3$ + $SiO_2$ + $TiO_2$/$Fe_2O_3$
Substratplättchen + $TiO_2$ + $SiO_2$ + $Fe_3O_4$
Substratplättchen + $TiO_2$ + $SiO_2$ + $Cr_2O_3$
Substratplättchen + $Cr_2O_3$
Substratplättchen + Ag
Substratplättchen + Au
Substratplättchen + $Fe_2O_3$ + $SiO_2$ + $TiO_2$/$Fe_2O_3$
Substratplättchen + $SiO_2$ + $TiO_2$ (Anatas)
Substratplättchen + $SiO_2$ + $SnO_2$ + $TiO_2$ (Rutil)
Substratplättchen + $SiO_2$ + $TiO_2$ + Erdalkalititanat
Substratplättchen + $SiO_2$ + $SnO_2$ + $TiO_2$ + Erdalkalititanat
Substratplättchen + $SiO_2$ + $TiO_2$ + $Fe_2O_3$
Substratplättchen + $SiO_2$ + $SnO_2$ + $TiO_2$ + $Fe_2O_3$
Substratplättchen + $SiO_2$ + $TiO_2$ + $Fe_3O_4$
Substratplättchen + $SiO_2$ + $SnO_2$ + $TiO_2$ + $Fe_3O_4$
Substratplättchen + $SiO_2$ + $TiO_2$ + $Cr_2O_3$
Substratplättchen + $SiO_2$ + $SnO_2$ + $TiO_2$ + $Cr_2O_3$
Substratplättchen + $SiO_2$ + $TiO_2$ + Karminrot
Substratplättchen + $SiO_2$ + $SnO_2$ + $TiO_2$ + Karminrot
Substratplättchen + $SiO_2$ + $TiO_2$ + Berliner Blau
Substratplättchen + $SiO_2$ + $SnO_2$ + $TiO_2$ + Berliner Blau
Substratplättchen + $SiO_2$ + $TiO_2$ / $Fe_2O_3$
Substratplättchen + $SiO_2$ + $SnO_2$ + $TiO_2$ / $Fe_2O_3$
Substratplättchen + $SiO_2$ + $TiO_2$ + $SiO_2$ + $TiO_2$
Substratplättchen + $SiO_2$ + $SnO_2$ + $TiO_2$ + $SiO_2$ + $SnO_2$ + $TiO_2$
Substratplättchen + $SiO_2$ + $Fe_2O_3$
Substratplättchen + $SiO_2$ + $Fe_3O_4$
Substratplättchen + $SiO_2$ + $TiO_2$ + $SiO_2$ + $TiO_2$/$Fe_2O_3$
Substratplättchen + $SiO_2$ + $SnO_2$ + $TiO_2$ + $SiO_2$ + $TiO_2$/$Fe_2O_3$
Substratplättchen + $SiO_2$ + $TiO_2$ + $SiO_2$ + $TiO_2$ + $TiO_2$/$Fe_2O_3$
Substratplättchen + $SiO_2$ + $SnO_2$ + $TiO_2$ + $SiO_2$ + $TiO_2$ + $TiO_2$/$Fe_2O_3$
Substratplättchen + $SiO_2$ + $SnO_2$ + $TiO_2$ + $SiO_2$ + $SnO_2$ + $TiO_2$ + $TiO_2$/$Fe_2O_3$
Substratplättchen + $SiO_2$ + $TiO_2$/$Fe_2O_3$ + $SiO_2$ + $TiO_2$ + $TiO_2$/$Fe_2O_3$
Substratplättchen + $SiO_2$ + $TiO_2$/$Fe_2O_3$ + $SiO_2$ + $SnO_2$ + $TiO_2$ + $TiO_2$/$Fe_2O_3$
Substratplättchen + $SiO_2$ + $TiO_2$/$Fe_2O_3$ + $SiO_2$ + $TiO_2$

Substratplättchen + $SiO_2$ + $TiO_2/Fe_2O_3$ + $SiO_2$ + $SnO_2$ + $TiO_2$
Substratplättchen + $SiO_2$ + $TiO_2/Fe_2O_3$ + $SiO_2$ + $TiO_2/Fe_2O_3$
Substratplättchen + $SiO_2$ + $TiO_2$ + $SiO_2$ + $TiO_2/Fe_2O_3$
Substratplättchen + $SiO_2$ + $TiO_2$ + $SiO_2$ + $Fe_3O_4$
Substratplättchen + $SiO_2$ + $TiO_2$ + $SiO_2$ + $Cr_2O_3$
Substratplättchen + $SiO_2$ + $Cr_2O_3$
Substratplättchen + $SiO_2$ + Ag
Substratplättchen + $SiO_2$ + Au
Substratplättchen + $SiO_2$ + $Fe_2O_3$ + $SiO_2$ + $TiO_2/Fe_2O_3$
Substratplättchen + $TiO_2$ (Anatas) + $SiO_2$
Substratplättchen + $SnO_2$ + $TiO_2$ (Rutil) + $SiO_2$.

15. Effektpigment nach einem oder mehreren der Ansprüche 1 bis 14, **dadurch gekennzeichnet, dass** es zur Erhöhung der Licht-, Wasser- und Wetterstabilität zusätzlich mit einer organischen und/oder anorganischen Schicht nachbehandelt wird.

16. Verfahren zur Herstellung des Effektpigments nach einem oder mehreren der Ansprüche 1 bis 15, **dadurch gekennzeichnet, dass** Substrate, die in Form von größeren Brocken oder groben Flocken vorliegen, zunächst gemahlen werden, anschließend die entstehenden Substratplättchen mit einem Durchmesser von 50-200 μm in ein Zerkleinerungsaggregat gegeben und mit Wasser und/oder einem organischen Lösemittel versetzt werden, und die so entstehende Suspension über mehrere Stunden in dem Zerkleinerungsaggregat mechanisch behandelt wird, wobei gleichzeitig die Oberfläche der Plättchen glatt poliert wird und die mechanische Belastung der Substratteilchen während dieses Schrittes dabei so gewählt wird, dass eine permanente Scherung zur weiteren schonenden Delaminierung von Teilchen und zur Glättung der Kanten und Oberflächen führt und eine enge Teilchengrößenverteilung durch einen anschließenden Klassierschritt in Form von mehreren Sedimentationsstufen erreicht wird.

17. Verfahren nach Anspruch 16, **dadurch gekennzeichnet, dass** die Teilchengrößenverteilung 5-60 μm beträgt.

18. Verwendung des Effektpigments nach einem oder mehreren der Ansprüche 1 bis 15 in Farben, Autolacken, Industrielacken, Lacken, Pulverlacken, Druckfarben, Kunststoffen, Knopfpasten, keramischen Materialien, Gläsern, zur Saatguteinfärbung, als Absorber bei der Lasermarkierung von Kunststoffen, Gläsern, Pappen und Papieren, als Absorber beim Laserschweißen von Kunststoffen, zur Farbgebung von Lebensmittel- und Pharmaprodukten, zur Farbgebung (Coatings) von Überzügen von Lebensmittel- und Pharmaprodukten, in kosmetischen Formulierungen, zur Herstellung von Pigmentpräparationen und Trockenpräparaten und in fälschungssicheren Wertdokumenten.

19. Formulierungen enthaltend ein oder mehrere Effektpigmente nach einem oder mehreren der Ansprüche 1 bis 15.

20. Formulierungen nach Anspruch 19, **dadurch gekennzeichnet, dass** sie neben den Effektpigmenten mindestens einen Bestandteil ausgewählt aus der Gruppe der Absorptionsmittel, Adstringenzien, antimikrobiellen Stoffe, Antioxidantien, Antiperspirantien, Antischaummitteln, Antischuppenwirkstoffe, Antistatika, Bindemittel, biologischen Zusatzstoffen, Bleichmitteln, Chelatbildnern, Desodorierungsmitteln, Emollentien, Emulgatoren, Emulsionsstabilisatoren, Farbstoffen, Feuchthaltemitteln, Filmbildnern, Füllstoffen, Geruchsstoffen, Geschmacksstoffen, Insect Repellents, Konservierungsmitteln, Korrosionsschutzmitteln, kosmetischen Ölen, Lösungsmitteln, Oxidationsmitteln, pflanzlichen Bestandteilen, Puffersubstanzen, Reduktionsmitteln, Tensiden, Treibgasen, Trübungsmitteln, UV-Filtern und UV-Absorbern, Vergällungsmitteln, Viskositätsreglern, Parfüm und Vitaminen enthalten.

21. Pigmentpräparationen enthaltend ein oder mehrere Bindemittel, optional ein oder mehrere Additive und Effektpigmente nach einem oder mehreren der Ansprüche 1 bis 15.

22. Trockenpräparationen enthaltend Effektpigmente nach einem oder mehreren der Ansprüche 1 bis 15.

Abbildung 1

Abbildung 2

Abbildung 3

Europäisches
Patentamt

European
Patent Office

Office européen
des brevets

**EUROPÄISCHER RECHERCHENBERICHT**

Nummer der Anmeldung

EP 12 00 7941

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | DE 10 2007 041027 A1 (ECKART GMBH [DE]) 5. März 2009 (2009-03-05) * Absatz [0157] - Absatz [0160] * ----- | 1-22 | INV. C09C1/00 |
| A | DE 10 2004 032799 A1 (MERCK PATENT GMBH [DE]) 17. Februar 2005 (2005-02-17) * Absätze [0010], [0011], [0015], [0016]; Anspruch 1 * ----- | 1-22 | |
| A | WO 2006/082415 A2 (DUNWILCO 1198 LTD [GB]; WHEELER IAN ROBERT [GB]) 10. August 2006 (2006-08-10) * Seite 17, Zeile 11 - Seite 18, Zeile 36 * ----- | 1-22 | |
| A | DE 103 29 780 A1 (MERCK PATENT GMBH [DE]) 7. Oktober 2004 (2004-10-07) * Absatz [0011]; Anspruch 4 * ----- | 1-22 | |

**RECHERCHIERTE SACHGEBIETE (IPC)**

C09C

Der vorliegende Recherchenbericht wurde für alle Patentansprüche erstellt

| Recherchenort | Abschlußdatum der Recherche | Prüfer |
|---|---|---|
| Den Haag | 13. März 2013 | Siebel, Eric |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 12 00 7941

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-03-2013

| Im Recherchenbericht angeführtes Patentdokument | Datum der Veröffentlichung | Mitglied(er) der Patentfamilie | Datum der Veröffentlichung |
|---|---|---|---|
| DE 102007041027 A1 | 05-03-2009 | DE 102007041027 A1<br>EP 2185654 A2<br>US 2010298469 A1<br>WO 2009030293 A2 | 05-03-2009<br>19-05-2010<br>25-11-2010<br>12-03-2009 |
| DE 102004032799 A1 | 17-02-2005 | DE 102004032799 A1<br>JP 2005042112 A<br>US 2005019575 A1 | 17-02-2005<br>17-02-2005<br>27-01-2005 |
| WO 2006082415 A2 | 10-08-2006 | AT 483766 T<br>AU 2006210733 A1<br>BR PI0607100 A2<br>CA 2596746 A1<br>CN 101137723 A<br>DK 1856211 T3<br>EP 1856211 A2<br>ES 2352750 T3<br>JP 2008531242 A<br>KR 20070106539 A<br>PT 1856211 E<br>US 2008134940 A1<br>US 2011297887 A1<br>WO 2006082415 A2 | 15-10-2010<br>10-08-2006<br>09-03-2010<br>10-08-2006<br>05-03-2008<br>24-01-2011<br>21-11-2007<br>22-02-2011<br>14-08-2008<br>01-11-2007<br>03-01-2011<br>12-06-2008<br>08-12-2011<br>10-08-2006 |
| DE 10329780 A1 | 07-10-2004 | CN 101695466 A<br>DE 10329780 A1 | 21-04-2010<br>07-10-2004 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1865032 A2
- US 5433779 A
- US 4038099 A
- US 6626989 B
- DE 2522572 C2
- EP 0271767 B1
- DE 1467468
- DE 1959988
- DE 2009566
- DE 2214545
- DE 2215191
- DE 2244298
- DE 2313331
- DE 2522572
- DE 3137808
- DE 3137809
- DE 3151343
- DE 3151354
- DE 3151355
- DE 3211602
- DE 3235017
- EP 0045851 A1
- EP 0106235 A1
- DE PS2215191 C
- DE OS3151354 A
- DE OS3235017 A
- DE OS3334598 A
- EP 0090259 A
- EP 0634459 A
- WO 9957204 A
- WO 9632446 A
- US 5759255 A
- US 5571851 A
- WO 0192425 A
- WO 2006021386 A1

**In der Beschreibung aufgeführte Nicht-Patentliteratur**

- **J.J. PONJEÉ.** *Philips Technical Review,* vol. 44 (3), 81 ff
- **P.H. HARDING ; J.C. BERG.** *J. Adhesion Sci. Technol.,* vol. 11 (4), 471-493